# FASCICULE DE BREVET EUROPEEN

(11) **EP 1 222 250 B1**
(45) Date de publication et mention de la délivrance du brevet: **13.06.2007**
(21) Numéro de dépôt: 00971462.7
(22) Date de dépôt: 13.10.2000
(51) Int. Cl.: C12N 1/20, C12Q 1/68

(54) **BACTERIES LACTIQUES MUTANTES CAPABLES DE SUREXPRIMER AU MOINS UNE PEPTIDASE**
MUTANTEN VON MILCHSÄUREBAKTERIEN DIE MINDESTENS EINE PEPTIDASE ÜBEREXPRIMEREN
MUTANT LACTIC BACTERIA WITH A CAPACITY FOR OVEREXPRESSING AT LEAST ONE PEPTIDASE

(30) Priorité: 15.10.1999 FR 9912924
(43) Date de publication de la demande: 17.07.2002
(73) Titulaire: INSTITUT NATIONAL DE LA RECHERCHE AGRONOMIQUE (INRA), 75338 Paris Cédex 07 (FR)
(72) Inventeur: GUEDON, Eric, 92100 Boulogne-Billancourt (FR); ANBA-MONDOLONI, Jamila, F-78180 Montigny-le-Bretonneux (FR); DELORME, Christine, F-91120 Palaiseau (FR); RENAULT, Pierre, F-78180 Montigny-le-Bretonneux (FR)
(74) Mandataire: Breese, Pierre
(86) Numéro de dépôt international: PCT/FR2000/002869
(87) Numéro de publication internationale: WO 2001/029183

(56) Documents cités:
- KUNJI EDMUND R S ET AL: "Transport of beta-Casein-derived Peptides by the Oligopeptide Transport System Is a Crucial Step in the Proteolytic Pathway of Lactococcus lactis." JOURNAL OF BIOLOGICAL CHEMISTRY, vol. 270, no. 4, 1995, pages 1569-1574, XP002141676 ISSN: 0021-9258
- MARUGG JOEY D ET AL: "Medium-dependent regulation of proteinase gene expression in Lactococcus lactis: Control of transcription initiation by specific dipeptides." JOURNAL OF BACTERIOLOGY, vol. 177, no. 11, 1995, pages 2982-2989, XP002141677 ISSN: 0021-9193 cité dans la demande
- FISHER SUSAN H ET AL: "Role of CodY in regulation of the Bacillus subtilis hut operon." JOURNAL OF BACTERIOLOGY, vol. 178, no. 13, 1996, pages 3779-3784, XP002160377 ISSN: 0021-9193
- DATABASE BIOSIS [en ligne] BIOSCIENCES INFORMATION SERVICE, PHILADELPHIA, PA, US; avril 1999 (1999-04) FISHER SUSAN H: "Regulation of nitrogen metabolism in Bacillus subtilis: Vive la difference!" Database accession no. PREV199900269050 XP002160389 & MOLECULAR MICROBIOLOGY, vol. 32, no. 2, avril 1999 (1999-04), pages 223-232, ISSN: 0950-382X
- KUNJI E ET AL: "The proteolytic systems of lactic acid bacteria" ANTONIE VAN LEEUWENHOEK, vol. 70, 1996, pages 187-221, XP000914826 cité dans la demande

## Description

La présente invention concerne des mutants de bactéries lactiques, comme *Lactoccocus lactis,* capables de surexprimer au moins une et de préférence plusieurs peptidases. Ces mutants présentent des activités peptidolytiques fortes qui permettent d'accélérer la dégradation de la caséine en acides aminés. Ces mutants sont donc tout particulièrement utiles pour augmenter la vitesse d'affinage des fromages car les acides aminés sont des précurseurs dans la synthèse d'arômes. L'invention concerne également une méthode d'identification de ces mutants et les constructions génétiques pour la mise en oeuvre de cette méthode. L'invention concerne enfin l'utilisation de ces bactéries mutantes dans un procédé de fabrication et/ou de maturation de fromages.

*Lactococcus lactis* possède un système protéolytique complexe pour dégrader les protéines du lait et en particulier la caséine. La caséine est la protéine majoritaire du lait qui fournit tous les acides aminés nécessaires à la croissance (12). La caséine est dégradée en oligopeptides par une protéase de paroi. Ces oligopeptides entrent dans la cellule par des systèmes de transport spécifiques puis sont hydrolysés en acides aminés à l'intérieur de la cellule par des peptidases (14). En plus de leur rôle dans la nutrition azotée de *L. lactis,* les peptidases pourraient avoir également un rôle important dans le développement des flaveurs lors de l'affinage de certains fromages.

Dix gènes de peptidases ont été clonés et leurs produits caractérisés biochimiquement chez *L. lactis.* Elles sont regroupées dans différentes classes suivant la position et la nature de la liaison peptidique qu'elles hydrolysent et ont souvent une spécificité large (14). A l'heure actuelle, peu d'études sur la régulation de l'expression de ces peptidases chez *L. lactis* ont été menées et seule la régulation de la protéase de paroi a été étudiée de manière approfondie (18, 19).

Or, l'expression de certains gènes chez *Lactococcus lactis* peut être critique lors de procédés de fabrication de fromages. Aussi, les Inventeurs ont considéré que l'évaluation du niveau d'expression de ces gènes peut se faire en déterminant l'efficacité de leurs promoteurs car la transcription est un des paramètres qui contrôle l'expression des gènes. Les Inventeurs ont donc développé dans le cadre de la présente invention des outils basés sur l'utilisation de gènes rapporteurs. Ils ont construit des vecteurs adaptés à l'étude systématique de nombreux promoteurs dans différents contextes cellulaires et environnementaux et qui peuvent être transférés aisément dans un grand nombre de souches de *L. lactis.* Ces vecteurs ont été utilisés pour étudier la variabilité de l'expression des enzymes du système protéolytique de *L. lactis.* L'expression de seize gènes codant pour des enzymes impliquées dans la protéolyse de la souche de *L. lactis* subsp. *cremoris* MG1363 ainsi que les deux gènes de la protéase de paroi des souches WG2 et SK11 a pu ainsi être caractérisée, soit grâce aux vecteurs développés par les Inventeurs, soit grâce à la détection des ARN messagers par la technique de Northern-blot (8).

Les travaux réalisés dans le cadre de la présente invention sur la caractérisation de l'expression des gènes codant pour des peptidases, des protéases et des protéines de transport de *L. lactis* ont permis de mettre en évidence une régulation coordonnée de leur expression et ainsi de déterminer les facteurs pouvant affecter cette expression. Les Inventeurs ont notamment réalisé une étude systématique de la transcription des seize gènes ci-dessus impliqués dans la protéolyse.

Il a ainsi été montré que la transcription de 8 des seize gènes testés est régulée et réprimée simultanément par des dipeptides via le pool intracellulaire d'acides aminés branchés : isoleucine, leucine et valine. Il s'agit des promoteurs des gènes des peptidases suivants :
- *prtP,* qui est la protéase de paroi (14), et plus particulièrement *prtPWG* qui est une protéase de paroi isolée de la souche WG2, et *prtPSK11,* qui est une protéase de paroi isolée de la souche SK11,
- *pepN* et *pepC,* qui sont les aminopeptidases de spécificité générale majeure dans la cellule (14),
- *pepO,* qui est une endopeptidase impliquée dans la dégradation des oligopeptides (14),
- *opp,* qui est l'opéron codant pour le système d'entrée des oligopeptides (14),
- *dtpt,* qui code pour une protéine de transport des di et tripeptides hydrophiles (14),
- *pepDA2,* qui code pour une dipeptidase générale.

Il a aussi été montré dans le cadre des travaux ayant conduit à la présente invention que la transcription des gènes du système protéolytique chez *L. lactis* est régulée par les produits de divers gènes. On peut citer tout particulièrement le gène *codY* qui constitue un régulateur central réprimant la transcription des gènes du système protéolytique chez *L. lactis.* On peut encore citer l'un des gènes de l'opéron *lev* et un gène codant pour une β-glucosidase. Concernant le gène codY, les seules études qui ont été initiées à l'heure actuelle sont des études sur le rôle du gène codY dans la régulation d'opérons de *Bacillus subtilis* et notamment de l'opéron *hut* (23).

Les Inventeurs ont donc mis en oeuvre une stratégie de mutagenèse aléatoire, appliquée à la souche MG1363 (21), pour trouver des régulateurs de la transcription de ces peptidases. La fusion du second promoteur de l'opéron *opp-pepO* (*PpepOA*) au gène de la β-galactosidase a servi de rapporteur pour visualiser des mutants dont la transcription de ce promoteur est dérégulée. Les inventeurs ont ainsi isolé des mutants de *L*. *lactis,* obtenus par insertion d'un transposon.

La présente invention a donc pour objet des mutants de bactéries lactiques capables de surexprimer une ou plusieurs peptidases, caractérisés en ce que le gène *codY* est inactivé.

Il peut s'agir d'une inactivation totale ou partielle. On entend par inactivation totale, le fait que ledit gène *codY* n'est pas du tout exprimé, et par inactivation partielle, le fait que ledit gène *codY* est encore exprimé mais pas suffisamment pour observer l'effet de régulation négative rencontré chez une bactérie non mutée.

Une première forme de réalisation d'une telle inactivation consiste en une modification de la séquence d'ADN du gène *codY* ou de la séquence du promoteur du gène *codY.* Une seconde forme de réalisation d'une telle inactivation consiste en une modification du gène dtpT codant pour un système de transport intracelllulaire du gène *codY* et de protéine cofacteurs du gène *codY.*

A titre de bactéries lactiques mutantes selon l'invention, on peut citer plus particulièrement des mutants de *L. lactis* et de *S*. *thermophilus.*

On entend tout particulièrement par mutants de bactéries lactiques selon l'invention, des bactéries génétiquement modifiées de façon à ce que le gène *codY* est inactivé totalement ou partiellement. Les mutants de bactéries lactiques selon l'invention sont donc avantageusement obtenus par mutagenèse.

L'invention concerne plus particulièrement des mutants de bactéries lactiques et notamment de *Lactoccocus lactis* dont le gène *codY,* commun à deux au moins et de_ préférence trois des promoteurs des gènes de peptidases, est inactivé.

Les promoteurs des gènes de peptidases dont le facteurs de régulation négative de la transcription, *codY,* est inactivé, sont par exemple choisis parmi les gènes *prtP, pepN, pepC, pepX, pepO, pepDA2, dtpT* et l'opéron *opp.*

Il sera fait référence dans ce qui suit à la liste de séquences en annexe dans laquelle :
- SEQ ID No. 1 représente la séquence du gène *codY* de *L. lactis* MG1363.
- SEQ ID No. 2 représente la séquence du gène *dtpT L. lactis* MG1363.
- SEQ ID No. 3 représente la séquence du gène *secA* de *L. lactis* IL1403.
- SEQ ID No. 4 représente la séquence du gène *secY* de *L. lactis* IL1403.
- SEQ ID No. 8 représente une partie de la séquence du gène *codY* de *S. thermophilus.*
- SEQ ID No. 9 représente la séquence complète du gène *codY* de *S. thermophilus.*

Un premier facteur de régulation négative des peptidases de bactéries, lactiques notamment de *L. lactis* identifié par les Inventeurs est constitué par le pool intracellulaire d'acides aminés branchés qui répriment la transcription de plusieurs gènes de peptidase. Un premier type de mutants est donc caractérisé par la modification de ce pool intracellulaire d'acides aminés branchés. On entend de préférence par modification, une diminution de la quantité d'acides aminés branchés. Un exemple de modification du pool d'acides aminés branchés consiste à modifier sélectivement leur entrée dans la cellule, notamment en bloquant l'un au moins des systèmes de transport :
- des acides aminés,
- des di- et tripeptides,
- des oligopeptides.

Des mutants d'un système de transport des dipeptides et tripeptides ont été obtenus par mutagenèse aléatoire dans le gène *dtpT* (10) de *L. lactis* dont la séquence est donnée dans la liste de séquence en annexe sous le numéro SEQ ID No. 2. Les travaux réalisés dans l'art antérieur sur ce gène n'ont jamais mis en évidence qu'il pouvait s'agir de mutants de *Lactoccocus lactis* capables de surexprimer une ou plusieurs peptidases. Un exemple de gène *dtpT* modifié dans un mutant est caractérisé par l'insertion par exemple du plasmide *pGhost-IIS1* après les nucléotides en positions 280 et 470 dans la séquence SEQ ID No. 2.

La répression de la transcription des gènes codant pour les peptidases est levée chez les mutants de l'invention et peut résulter comme indiqué précédemment de la variation du pool intracellulaire des acides aminés branchés. La variation du pool intracellulaire des acides aminés branchés peut également provenir de la variation de la dégradation desdits peptides (di, tri ou oligopeptides). En conséquence, des mutants de bactéries lactiques et plus particulièrement de *L. lactis* sont des mutants dans lesquels l'un au moins des gènes codant les peptidases responsables de la dégradation de ces dipeptides, tripeptides ou oligopeptides est inactivé.

Un facteur important de régulation négative identifié par les inventeurs est le produit du gène *codY* qui réprime la transcription de plusieurs peptidases au niveau de leur promoteur. En effet, les inventeurs ont obtenu par mutagenèse des mutants de *L. lactis* inactivés dans un gène qui est homologue au gène *codY* de *Bacillus subtilis.* Chez un mutant *codY* reconstruit par les inventeurs par mutagenèse dirigée, il a été observé que la transcription du gène *pepOA* et la transcription d'au moins trois gènes de peptidases ne sont plus réprimés par les dipeptides. Ainsi, l'inactivation du gène *codY* chez *L. lactis* permet d'augmenter l'expression des gènes de plusieurs peptidases d'un facteur 4 à 55 dans un milieu contenant une source de peptides qui normalement les répriment dans la souche sauvage.

La séquence d'ADN du gène *codY* de *L. lactis* et de la séquence de la protéine codY pour lequel il code sont représentées dans SEQ ID No. 1 en annexe.

Les inventeurs ont également mis en évidence la présence du gène *codY* chez *S*. *thermophilus,* qui est comme *L. Lactis* une bactérie lactique. La séquence d'ADN partielle du gène *codY* de *S*. *thermophilus* et la séquence de la protéine pour lequel il code sont représentées dans SEQ ID No. 8 en annexe. La séquence d'ADN complète du gène *codY* de *S. thermophilus* et la séquence de la protéine pour lequel il code sont représentées dans SEQ ID No. 9 en annexe. Ainsi l'inactivation complète ou partielle du gène *codY* chez les autres bactéries lactiques (Streptocoque, Lactobacille, Pediocoque, Leuconostoc) pourrait également permettre d'augmenter l'expression des peptidases.

En conséquence, un type préféré de mutants de bactéries lactiques selon l'invention, plus particulièrement de *L. lactis,* est caractérisé par l'inactivation du gène *codY.* Un premier exemple d'une telle inactivation consiste en une modification de la séquence d'ADN du gène *codY,* tout particulièrement des séquences SEQ ID No. 1, 8 ou 9 en annexe, ou de la séquence du promoteur du gène *codY.* Un second exemple d'une telle inactivation consiste en une modification du gène dtpT codant pour un système de transport intracellulaire du gène *codY* et de protéine cofacteurs du gène *codY.*

Comme indiqué précédemment, dans les bactéries mutantes pour *codY* de l'invention l'expression d'au moins 3 peptidases est augmentée de 4 à 55 fois dans un milieu contenant une source de peptides qui normalement répriment leur expression. Une bactérie mutante pour *codY* selon l'invention interrompt la cascade de régulation qui conduit à la répression des peptidases via le pool de peptides du milieu extérieur. Un changement de la séquence d'ADN dans le gène *codY* ou dans la séquence de son promoteur consiste par exemple en une mutation ou une délétion qui peuvent être réalisées par des méthodes bien connues de mutagenèse. Ainsi les inventeurs ont répertorié 13 mutants de codY avec par exemple une insertion du plasmide *pGhost-IIS1* après les nucléotides en positions 87, 112, 122, 289, 313, 409, 575, 604, 641, 693, 821, 877 et 882 dans la séquence SEQ ID No. 1.

Comme indiqué précédemment, d'autres mutants de bactéries lactiques, notamment de *L. lactis,* capables de surexprimer une ou plusieurs peptidases ont été caractérisés par les Inventeurs. A titre d'exemples de tels mutants, on peut citer les mutants dans lesquels un gène codant pour des protéines impliquées dans la sécrétion des protéines de transport des dipeptides ou tripeptides est inactivé. Il s'agit plus particulièrement de mutants dont l'un au moins des gènes *secA* (3) et *secY* (13) est modifié. Les protéines codées par ces gènes pourraient intervenir dans la translocation de la protéine DtpT qui est impliquée dans le transport des di- et tripeptides. Les séquences des gènes *seca* et *secY* de *L. lactis* sont données dans la liste de séquences en annexe respectivement sous les numéros SEQ ID No. 3 et SEQ ID No. 4. Des mutants pour *secA* ont été préparés par insertion du plasmide *pGhost-IIS1* après les nucléotides en positions 1689 et 1698 dans la séquence SEQ ID No. 3. Des mutants pour *secY* ont été préparés par insertion du plasmide *pGhost-IIS1* après les nucléotides en positions 1273 et 1281 dans la séquence SEQ ID No. 4.

L'invention concerne également l'utilisation des mutants de bactéries lactiques tels que décrits précédemment ou un mélange de ceux-ci dans un procédé de fabrication et/ou de maturation du fromage. De façon avantageuse, les mutants de bactéries lactiques tels que décrits précédemment ou un mélange de ceux-ci sont utilisés dans un procédé de fabrication et/ou de maturation de fromages type pâte molle ou pâte pressée.

D'autres avantages et caractéristiques de l'invention apparaîtront des exemples qui suivent. Ces exemples concernent l'obtention par mutagenèse de mutants de *L. lactis* selon l'invention, et se réfèrent aux figures en annexe dans lesquelles :
- la figure 1 représente l'activité luciférase de différentes fusions transcriptionnelles à D.O. 0,4 en milieu chimiquement défini (CDM) acides aminés (AA) et CDM casitone (Cas),

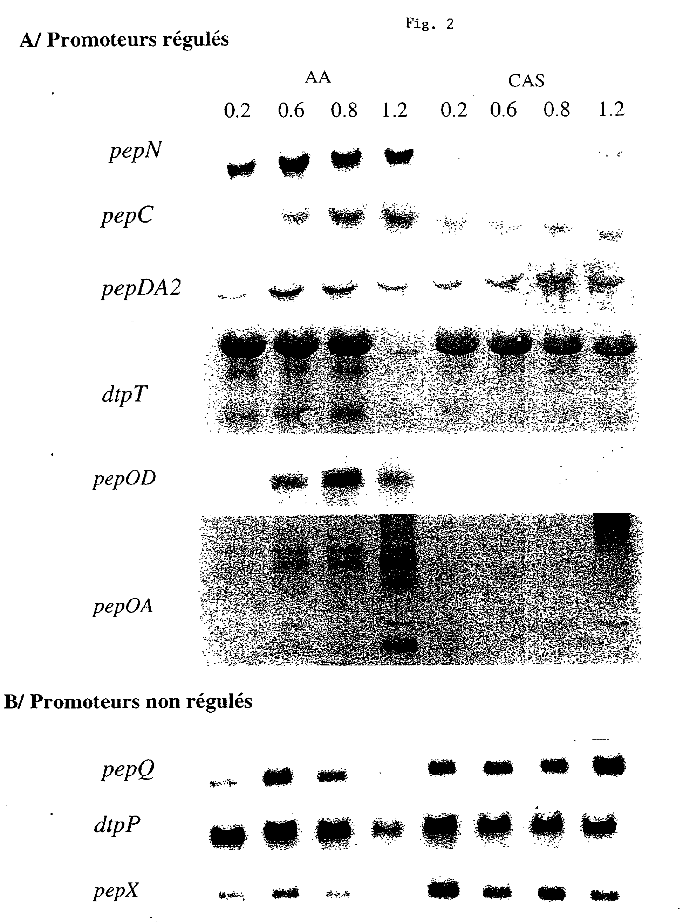

- la figure 2 représente la répression de la transcription en A des promoteurs régulés, et en B des promoteurs non régulés. Ces résultats ont été obtenus à partir de l'extraction des ARNm totaux de la souche sauvage cultivée en CDM + acides aminés (AA) et CDM + casitone (Cas) à différentes densités optiques (0,2; 0,6; 0,8; 1,2). Les hybridations ont été effectuées avec différentes sondes spécifiques des promoteurs de peptidases. Les promoteurs régulés correspondent à une diminution de l'ARN dans les conditions de croissance en présence de casitone, ce qui est le reflet d'une répression de la transcription.

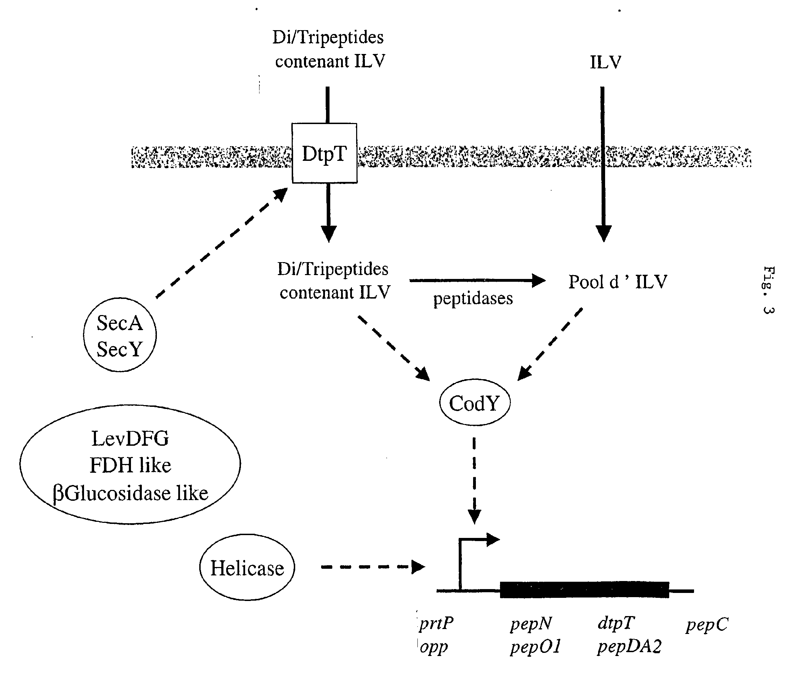

- La figure 3 est une représentation schématique des différents facteurs intervenant sur l'expression des peptidases de *L. lactis* et ayant permis de concevoir les différents mutants de l'invention.

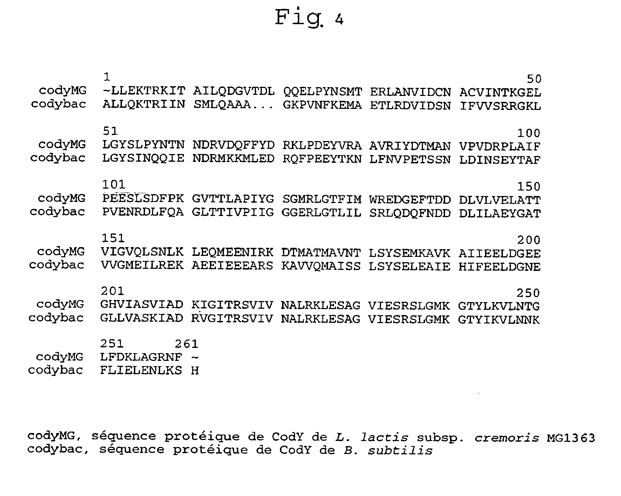

- La figure 4 est un alignement des séquences des gènes *codY* de *L. lactis* et de *Bacillus subtillis.*

### I - Matériel et Méthodes.

### 1) Souches bactériennes, milieux, vecteurs et manipulations d'ADN.

La souche de *L. lactis* MG1363 a été cultivée à 30°C en milieu M17 glucose. Au besoin, 5µg/ml d'érythromycine sont ajoutés au milieu de culture. Les promoteurs de peptidases à étudier (PepP, PepA, PepF2, PepDA1, PepOA, PepQ, PepX, PepOD, PepM, PepT, PepN et PepC) et les deux promoteurs de l'opéron *opp* (*pepoA*/*pepoD*) ont été amplifiés par PCR à l'aide d'amorces spécifiques à partir du chromosome de la souche de *L. lactis* subsp. *cremoris* MG1363. Une série de vecteurs à réplication conditionnelle, contenant les gènes rapporteurs de la luciférase de *Vibro harveyi* et un fragment du facteur sexuel (gène *cluA*) a été construite. Le pVar-1 a été utilisé pour fusionner aux gènes luciférase, les fragments d'ADN obtenus par PCR et correspondant aux différents promoteurs.

### 2) Intégration des fusions transcriptionnelles sur le chromosome de MG1363 et conjugaison.

Après transformation de la souche MG1363 par les plasmides pVar-1 contenant les promoteurs de peptidases, les fusions sont intégrées dans le chromosome par recombinaison homologue, soit au locus promoteur peptidase, soit au locus du facteur sexuel (dans le gène *cluA*)*.* L'identification du locus d'intégration se fait grâce à des amorces appropriées par amplification PCR et par hybridation d'un gel Southern. Le transfert du facteur sexuel se fait par conjugaison entre deux souches (5).

### 3) Détermination de l'activité luciférase chez L. lactis.

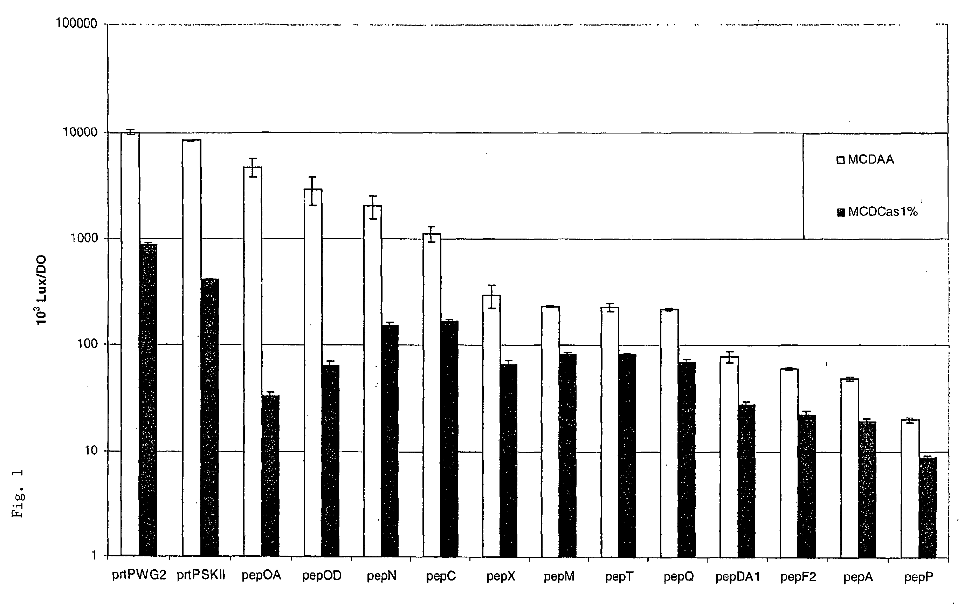

Les mesures d'activité luciférase sont effectuées sur le luminomètre Bertold Lumat LB9501. Un millilitre de culture de *L. lactis* est mélangé avec 5µl de nonaldehyde et l'émission de lumière est directement mesurée. La valeur du pic est ramenée à la DO₆₀₀ₙₘ de la culture et l'activité luciférase est mesurée tout au long de la croissance. L'activité luciférase reportée dans la figure 1 est mesurée à DO₆₀₀ₙₘ = 0,4 et exprimée en 10³ lux/DO.

### 4) Milieu Chimiquement Défini (CDM).

Ce milieu chimiquement défini (CDM) est décrit dans Sissler et al. (22). La source d'azote de ce milieu est un mélange d'acides aminés. Dans le "CDM cas", est ajouté un extrait de casitones (caséines du lait dégradées par des enzymes pancréatiques) qui est une source de petits peptides.

### 5) Constructions: PpepOA-βgal.

Le second promoteur de l'opéron *opp-pepO* (PpepOA) de la souche MG1363 a été amplifié par les oligonucléotides suivant (GGGAATTCTTTGGGAACAATGATAA et CGGGATCCGTTACTTCTGAACCA) et le fragment amplifié de 500pb a été cloné dans le plasmide pJIM762 au site *Eco*RI-*Bam*HI en amont du gène de la β-galactosidase de *Escherichia coli* (E. Guédon, résultats non encore publiés). Ce plasmide, dont le gène de la β-galactosidase est sous le contrôle des signaux d'expressions de PpepOA, a été intégré dans le chromosome de la MG1363 par recombinaison homologue au locus promoteur. La transcription à PpepOA est réprimée par les dipeptides contenu dans la casitone du milieu et la souche contenant la fusion est blanche sur un milieu chimiquement défini (CDM) contenant des casitones. La transcription à PpepOA est déréprimée sur un milieu CDM contenant des acides aminés comme source d'azote et la souche contenant la fusion est bleue [dans les deux cas la souche est cultivée avec du phospho-β-galactoside (P-β-gal).

### 6) Plasmide Pghost8-ISS1.

Ce plasmide à réplication conditionnelle (protéine de réplication thermosensible) possède un marqueur d'antibiotique tétracycline et une séquence d'insertion IS*S1* (16). L'augmentation de la température de 30°C à 37°C inhibe la réplication de ce plasmide et les souches résistantes à la tétracycline obtenues contiennent le plasmide intégré dans le chromosome. Il s'intègre de façon aléatoire dans le chromosome de *L. lactis* par transposition réplicative (16).

### 7) Mutagenèse par transposition.

Une mutagenèse aléatoire est effectuée avec le plasmide thermosensible pGhost8-IS*S1* dans la souche MG1363 contenant la fusion promoteur PpepOA-p-gal. En milieu MCD casitone, en présence de P-β-gal, sur 50000 clones blancs isolés, 46 présentent un phénotype de couleur bleue. Dans ces mutants l'expression de la fusion β-gal est déréprimée. Le plasmide pGhost8-IS*S1* est donc inséré dans un gène dont le produit est un répresseur direct ou indirect de l'expression de PpepOA.

### 8) Identification des mutants par clonage des jonctions.

La transposition par IS*S1* dans le chromosome donne une insertion du pGhost8 entouré d'une copie dupliquée de IS*S1*. Les jonctions ont été clonées, en utilisant des sites uniques de restriction (*Eco*RI et *Hin*dIII) présents sur le pGhost8. La digestion du chromosome par ces enzymes permet d'obtenir le plasmide pGhost8 contenant les régions flanquantes. Le site de transposition est ainsi caractérisé par séquençage des jonctions avec les oligonucléotides suivants (pour la jonction *EcoRI* : TCACCTCATATAAATTCCCCA et AAATGGAACGCTCTTCGG) (pour la jonction *HindIII* : CGCCAGGGTTTTCCCAGTCACGAC et ACCAACAGCGACAATAATCACA).

### 9) Mutant codY.

Une mutagenèse aléatoire a permis d'obtenir entre autre, des mutants *codY* pour lesquels la transcription de plusieurs gènes codant pour des enzymes protéolytiques est dérégulée. L'inactivation de ce gène chez *L. lactis* augmente l'expression des gènes *opp-pepO, pepN* et *pepC* respectivement d'un facteur 55, 14 et 4 en milieu CDM avec casitones où l'expression est normalement réprimée par la source de peptides.

### 10) Inactivation de codY par simple crossing-over.

Un fragment PCR de 540 pb a été amplifié par les oligonucléotides suivants (CAGTATGACTGAACGCTTGGC et GCGATAACATGCCCTTCTTCA) et cloné dans le plasmide pJIM2242. Ce plasmide est intégré dans le gène *codY* par simple crossing-over et un mutant *codY* est vérifié par une hybridation Southern. Ce mutant a le même phénotype que les mutants *codY* obtenus par mutagenèse.

### 11) Autres mutants.

La mutagenèse aléatoire a permis d'identifier plusieurs mutants autre que codY. Des mutations des gènes *dtpT,* et *secY* ont également conduit à des mutants surexprimant au moins *pepO* ou plusieurs peptidases.

### II - Résultats.

### 1) Construction du vecteur pVar-1.

Des vecteurs intégratifs permettant de suivre l'expression d'un gène rapporteur sous le contrôle d'un promoteur sur le chromosome ont été construits. Les gènes de la luciférase de *Vibrio harveyi* ont été utilisés comme gène rapporteur. L'activité luciférase des fusions transcriptionnelles avec les promoteurs de gènes de peptidases est le reflet de l'expression des gènes de peptidases (11, 20). Un vecteur qui se réplique de façon conditionnelle a été utilisé pour intégrer les fusions transcriptionnelles sur le chromosome (15). Ce vecteur a été conçu pour être facilement transférable par conjugaison, en particulier dans des souches industrielles difficilement transformables. Un fragment (gène *cluA*) de l'élément chromosomique de 60kb nommé le facteur sexuel, que possèdent certaines souches de lactocoques, a été introduit dans ce vecteur. Cet élément est capable de s'auto-transférer à une haute fréquence par conjugaison dans l'espèce *L. lactis* (7). En intégrant nos fusions transcriptionnelles dans ce facteur sexuel, celles-ci sont alors transférables à d'autres souches de lactocoques par conjugaison du facteur sexuel. Parmi différents vecteurs construits, le pVar-1 utilisé dans cette étude contient, en plus des composants décrits ci-dessus un gène de résistance à l'érythromycine. Il a été vérifié que les fusions transcriptionnelles intégrées au locus promoteur ou dans le gène *cluA* avec le pVar-1 avaient des activités luciférases identiques (9).

### 2) Expression de fusions avec les promoteurs des gènes codant pour des peptidases.

Les promoteurs de 11 gènes codant pour des peptidases (*pep*) de *L. lactis* MG1363 : *pepA, pepC, pepDA1, pepF2, pepM, pepn, pepP, pepQ, pepT, pepX ;* et les deux promoteurs de l'opéron *opp-pepO* (*PpepOA* et *PpepOD*) dans lequel se trouve le gène pepO, ont été clonés, fusionnés au gène *lux* dans le vecteur pVar-1 et intégrés par recombinaison homologue dans le chromosome de *L. lactis* MG1363 au locus des différents promoteurs. Les deux promoteurs de protéase (*prt* des souches WG2 et SK11) sont fusionnés au gène luciférase sur un plasmide. L'expression de ces fusions a été déterminée en milieu CDM et CDM cas et les valeurs des mesures luciférases sont rapportées dans la figure 1. En milieu CDM, selon le taux de luciférase mesuré, les fusions ont été regroupées en différentes classes. La plus forte activité luciférase est obtenue avec les fusions plasmidiques contenant les promoteurs des gènes *prt* (10.10³ lux/DO (10³)). Pour les fusions chromosomiques, la plus forte activité luciférase est obtenue avec les promoteurs PpepN, PpepC, PpepOA et PpepOD (1 à 5 10.10³ lux/DO (10³) une activité moyenne est obtenue avec les promoteurs des gènes *pepQ, pepX, pepM* et *pepT* (200 à 300 lux/DO (10³)) et une activité faible est obtenue avec les promoteurs des gènes *pepP, pepA, pepF2* et *pepDA1* (20 à 80 lux/DO (10³)). Il est à remarquer que les niveaux d'expression les plus élevés sont obtenus avec des fusions contenant les promoteurs des gènes codant pour les peptidases de très large spécificité (pepC, pepN, pepO) et pour un système de transport des oligopeptides (Opp) qui est essentiel à la croissance des lactocoques en milieu lait. L'expression des gènes de peptidases est diminuée en milieu CDM cas qui contient une source d'azote constituée d'acides aminés et de peptides (figure 1). La force des promoteurs *PpepP, PpepA, PpepF2, PpepDA1, PpepQ, PpepT* et *PpepM* est diminuée de 2 à 3 fois en CDM cas tandis que celle des promoteurs *PpepX, PpepC, PpepN, PprtPWG2, PprtPSK11, pepO,* et l'opéron *opp-pepO* est réprimée respectivement 5, 7, 13, 21, 12 et 153 fois par les dipeptides du milieu de culture via le pool d'acides aminés branchés dans la cellule. L'analyse des transcrits par Northern Blot a permis de confirmer les résultats obtenus avec les fusions transcriptionnelles et de montrer que la transcription des gènes *pepDA2* et *dtpT,* mais non celle de *dtpP,* était réprimée par les peptides de la casitone (figure 2).

### 3) Obtention et caractérisation de mutants déréprimés.

Sur un milieu riche en peptides et en présence de P-βgal, la souche sauvage contenant la fusion *PpepOA-*βgal donne des colonies blanches car l'expression de la fusion est réprimée. Les mutants obtenus donnent des colonies bleues car la fusion fusion *PpepOA*-βgal est déréprimée.

Différents gènes des mutants dans lesquels le pGhost s'est inséré ont été identifiés (*codY, dtpT, secA* et *secY*). L'analyse des ARNm par Northern Blot d'une souche mutante cultivée dans un milieu riche en peptides, a confirmé que la transcription du gène *pepO* n'est plus réprimée dans les mutants des gènes *codY* et *dtpT.* La dérepression de *pepO* dans les mutants des gènes codant pour SecA et SecY reste à être confirmé.

### 4) Caractérisation de l'expression des peptidases dans les mutants dérégulés.

La transcription des gènes de peptidases a été caractérisée dans les mutants par mesure d'activité. Deux classes de mutants peuvent être obtenues, l'une pour laquelle la transcription de plusieurs peptidases est déréprimée (mutants pléiotropes) et l'autre où seule la transcription du gène *pepO* est déréprimée.

Mutants des gènes *codY, dtpT* : dans un milieu riche M17 qui contient des peptides répresseurs, les activités luciférases ont été mesurées dans une souche sauvage et dans un mutant *codY* pour les promoteurs *PpepOD, PpepC* et *PpepN,* et dans un mutant *dtpT* pour le promoteur *PpepOD.* Dans un mutant *codY,* la répression de la transcription est diminuée d'un facteur 35, 4 et 14 respectivement pour les gènes *pepOD, pepC et pepN.* Dans un mutant *dtpT,* la répression de la transcription est diminuée d'un facteur 15 pour le gène *pepOD.*

### REFERENCES BIBLIOGRAPHIQUES

1) BOUTROU R., SEPULCHRE A., GRIPON J.C., MONNET V., 1998. Simple test for predicting the lytic behavior and proteolytic activity of lactococcal strains in cheese. J. Dairy Sci., 81,2321-2328.
2) DELORME C., EHRLICH S.D., RENAULT P., 1999. Regulation of expression of the Lactococcus lactis histidine operon. J. Bacteriol., 181, 2026-2037.
3) Driessen A., Fekkes P., van der Wolk JP, "The Sec system" 1998, Curr. Opin. Microbiol., 1(2), 216-222.
4) DROUAULT S., CORTHIER G., DELORME C., EHRLICH S.D., RENAULT P., 1998. Régulations métaboliques de Lactococcus lactis en culture pure ou mixte dans le lait. Lait, 77, 15-23.
5) GASSON M. J., SWINDELL S., MAEDA S., DODD H.M., 1992. Molecular rearrangement of lactose plasmid DNA associated with high-frequency transfer and cell aggregation in Lactococcus lactis 712. Mol. Microbiol., 6, 321-3-3223.
6) GODON J. J., JURY K., SHEARMAN C. A., GASSON M. J., 1994. The Lactococcus lactis sex-factor aggregation gene cluA. Mol. Microbiol., 12, 655-663.
7) GODON J. J., PILLIDGE C. J., JURY K., GASSON, M. J., 1996. Caractérisation d'un élément conjugatif original, le facteur sexuel de Lactococcus lactis 712. Lait, 76, 41-50.
8) Guédon E., Renault P., Ehrlich SD., Delorme C. "Environmental factors involved in the transcriptional regulation of 18 proteolysis components in Lactococci" soumis pour publication).
9) E. Guédon, P. Renault, SD Ehrlich et C.Delorme "Evaluation de la diversité de l'expression génétique chez les lactocoques : développement d'un outil et son application aux peptidases", accepté pour publication dans Science des aliments).
10) Hagting A. et al., 1994, "The di- and tri-peptide transport protein of Lactococcus lactis", J. Biol. Chem., 269, 11391-11399.
11) HILL P. J., REES C. E. D., WINSON M. K., STEWART, G. S. A. B., 1993. The application of lux genes. Biotechnol. Appl. Biochem., 17, 3-14.
12) JUILLARD V., LE BARS D., KUNJI E. R., KONINGS W. N., GRIPON J. C., RICHARD, J., 1995 . Oligopeptides are the main source of nitrogen for Lactococcus lactis during growth in milk. Appl. Environ. Microbiol., 61, 3024-3030.
13) Koivula T., Palva I., Hemila H., "Nucleotide sequence of the secY gene from Lactococcus lactis and identification of conserved regions by comparison of four secY proteins" 1991, FEBS Lett. 288: 114-8.
14) KUNJI E. R., MIERAU I., HAGTING A., POOLMAN B., KONINGS, W. N., 1996. The proteolytic systems of lactic acid bacteria. Antonie Van Leeuwenhoek, 70, 187-221.
15) LAW J., BUIST G., HAANDRIKMAN A., KOK J., VENEMA G., LEENHOUTS K., 1995. A system to generate chromosomal mutations in Lactococcus lactis which allows fast analysis of targeted genes. J. Bacteriol., 177, 7011-7018.
16) Maguin E., Prevost , Ehrlich SD, Gruss A., "Efficient insertional mutagenesis in lactococci and other Gram-positive bacteria", 1996, J. Bact. 178, 931-935.
17) Martin-Verstraete I., Debarbouillé M., Klier A., Rapoport G. "Levanase operon of Bacillus subtilis includes a fructose specific phosphotransferase system regulating the expression of the operon" 1990, J. Mol. Biol., 244, 657-671.
18) MARUGG J. D., MEIJER W., VAN KRANENBURG R., LAVERMAN P., BRUINENBERG P. G., DE VOS W. M., 1995. Medium-dependent regulation of proteinase gene expression in Lactococcus lactis, control of transcription initiation by spécifie dipeptides. J. Bacteriol., 177, 2982-2989.
19) MEIJER W., MARUGG J. D., HUGENHOLTZ J., 1996. Regulation of proteolytic enzyme activity in Lactococcus lactis. Appl. Environ. Microbiol., 62, 156-161.
20) RENAULT P., CORTHIER G., GOUPIL N., DELORME C., EHRLICH S.D., 1996. Plasmid vectors for gram-positive bacteria switching from high to low copy number. Gene. 183, 175-182.
21) PREVOST H., EHRLICH S.D., GRUSS A., 1996, Efficient insertional mutagenesis in lactococci and other gram-positive bacteria. J. Bacteriol., 178 : 931-935.
22) SISSLER M., DELORME C., BOND J., EHRLICH SD, RENAULT P., FRANCKLYN C,. 1999, "An aminoacyl-tRNA synthetase paralog with a catalytic role in histidine biosynthesis" PNAS, 96:8985-8990.
23) FISHER H. S., ROHRER K., FERSON A. E.,. 1996, "Role of Cod Y in Regulation of the Bacillus subtilis hut Operon" J. of Bacteriology, Vol. 178, No. 13:3779-3784.

## Revendications

1. Mutant de bactérie lactique capable de surexprimer une ou plusieurs peptidases, **caractérisé en ce que** le gène codY est inactivé.

2. Mutant de bactérie lactique selon la revendication 1, **caractérisé en ce que** ladite inactivation est totale ou partielle.

3. Mutant de bactérie lactique selon l'une des revendications 1 ou 2, **caractérisé en ce que** la séquence d'ADN du gène codY ou la séquence du promoteur du gène codY est modifiée.

4. Mutant de bactérie lactique selon l'une des revendications 1 ou 2, **caractérisé en ce que** l'inactivation du gène codY se fait par modification du gène dtpT codant pour un système de transport intracellulaire des di- et tri-peptides.

5. Mutant de bactérie lactique selon l'une quelconque des revendications précédentes, **caractérisé en ce que** la bactérie lactique est *L. lactis.*

6. Mutant de bactérie lactique selon l'une quelconque des revendications précédentes, **caractérisé en ce que** la bactérie lactique est *S. thermophilus.*

7. Utilisation des mutants de bactéries lactiques selon l'une quelconque des revendications 1 à 6, ou un mélange de ceux-ci dans un procédé de fabrication et/ou de maturation du fromage.

## Claims

1. Mutant lactic bacteria capable of over-expressing one or several peptidases, **characterised in that** the codY gene is inactivated.

2. Mutant lactic bacteria according to claim 1, **characterised in that** the said inactivation is total or partial.

3. Mutant lactic bacteria according to either claims 1 or 2, **characterised in that** the DNA sequence of the codY gene or the sequence of the codY gene promoter is modified.

4. Mutant lactic bacteria according to either claims 1 or 2, **characterised in that** the codY gene is inactivated by modifying the dtpT gene coding for an intracellular transport system of the di- and tri-peptides.

5. Mutant lactic bacteria according to any one of the previous claims, **characterised in that** the lactic bacterium is *L. lactis.*

6. Mutant lactic bacteria according to any one of the previous claims, **characterised in that** the lactic bacterium is *S. thermophilus.*

7. Use of mutant lactic bacteria according to any one of claims 1 to 6, or a mix of these mutant bacteria, in a cheese fabrication and / or maturation process.

## Patentansprüche

1. Milchsäurebakterienmutant, der eine oder mehrere Peptidasen überexprimieren kann, **dadurch gekennzeichnet, dass** das Gen codY deaktiviert ist.

2. Milchsäurebakterienmutant nach Anspruch 1, **dadurch gekennzeichnet**, das diese Deaktivierung vollständig oder teilweise ist.

3. Milchsäurebakterienmutant nach Anspruch 1 oder 2, **dadurch gekennzeichnet**, das die DNA-Sequenz des Gens codY oder die Sequenz des Promotors des Gens codY modifiziert ist.

4. Milchsäurebakterienmutant nach Ansprüche 1 oder 2, **dadurch gekennzeichnet, dass** die Deaktivierung des Gens codY durch Modifikation des für ein System des intrazellulären Transports der di- und tri-peptides kodierenden Gens dtpT, erfolgt.

5. Milchsäurebakterienmutant nach einem der vorhergehenden Ansprüche, **dadurch gekennzeichnet, dass** das Milchsäurebakterium *L. lactis* ist.

6. Milchsäurebakterienmutant nach einem der vorhergehenden Ansprüche, **dadurch gekennzeichnet, dass** das Milchsäurebakterium *S*. *thermophilus* ist.

7. Verwendung von Milchsäurebakterienmutanten nach einem der Ansprüche 1 bis 6 oder einer Mischung derselben in einem Verfahren zur Herstellung und/oder Reifung von Käse.
